# EUROPEAN PATENT APPLICATION

(11) **EP 1 939 298 A1**
(43) Date of publication of application: **02.07.2008**
(21) Application number: 07025182.2
(22) Date of filing: 28.12.2007
(51) Int. Cl.: C12N 15/82, A01H 5/02, C12N 9/10

(54) **A process to produce a plant which has petals comprising non-acylated anthocyanins and acylyted anthocyanins**

(30) Priority: 28.12.2006 JP 2006356440
(71) Applicant: Kirin Holdings Kabushiki Kaisha, Tokyo 104--8288 (JP)
(72) Inventor: Umemoto, Naoyuki, Sakura-shi Tochigi 329-1414 (JP); Takeshita, Daigaku, Sakura-shi Tochigi 329-1414 (JP)
(74) Representative: HOFFMANN EITLE

(57) **Abstract**

It is intended to obtain a flowering plant having a novel variegated flower color such as a bicolor or to provide means for detecting a transposon involved therein. The present invention provides a process to produce a plant by use of a mutation (mutant gene) of a gene encoding a novel acyltransferase protein and a process to detect the mutation.

## Description

### BACKGROUND OF THE INVENTION

### Field of the Invention

The present invention relates to a transposon inserted in a gene encoding a novel acyltransferase protein and to a process to produce, by use of the same, a plant which has petals comprising non-acylated anthocyanins which are anthocyanins free of acyl groups and acylated anthocyanins which are anthocyanins having acyl groups. Specifically, the present invention relates to a process to produce a plant which has petals comprising non-acylated anthocyanins and acylated anthocyanins, comprising introducing a mutant gene of a gene encoding a novel acyltransferase protein, which comprises the transposon inserted at an arbitrary position, into a plant free of the mutant gene, or deleting a whole or partial gene function in a plant having a gene encoding a novel acyltransferase protein, and to a process to detect the presence or absence of a mutation attributed to the transposon associated with the deletion or the like.

### Background Art

The most important property of flowering plants is a flower color. Among the flower colors, anthocyanins are deeply involved in pink, red, blue, and purple flower colors and are important. To obtain various flower colors, breeding has been performed so far by crossing, mutations, or genetic recombination. Particularly, bicolor varieties, which have two flower colors in one petal, have highly been prized. However, genes or genetic backgrounds thereof have not been elucidated at the genetic level.

Anthocyanins have been known to become molecules having various color tones as a result of modifications with various acyl groups. As their molecular mechanisms have been elucidated in recent years, the presence of plant BAHD acyltransferases having common sequences and a group of genes encoding them has been revealed (D'Auria, Curr. Opin. Plant Biol., 9, 331-340 (2006)).

Pigments in the petals of carnations, one of three major horticultural flowering plants, are usually anthocyanins having acyl groups (M. Nakayama et al., Phytochemistry, 55, 937-939 (2000)). This acyl group in carnations is characterized by being modified with malic acid, unlike those in other plants. Varieties free of acyl groups (e.g., 'Nazareno', Kirin Agribio Co., Ltd.) have been bred in recent years, and a group of carnations having obviously different flower colors from ordinary carnation colors have been produced (M. Yamaguchi, Report of Research of Faculty of Horticulture, Minami Kyushu University, 19, 1-78 (1989); H. Yoshida, Journal of Horticulture (Engeigaku Zasshi in Japanese), 72 suppl. 1, 130 (2003)). However, molecular mechanisms to produce such unique flower colors of carnations have not been elucidated yet.

An object of the present invention is to provide a flowering plant having a novel variegated flower color by expressing or repressing a novel acyltransferase protein in petals by use of a mutant gene of a gene encoding the novel acyltransferase protein, which comprises a transposon, and to provide a process to detect the presence or absence of a mutation attributed to the transposon in a plant.

### SUMMARY OF THE INVENTION

The present inventors have conducted diligent studies on molecular mechanisms to produce the unique flower colors of carnations. As a result, the present inventors have newly found a gene encoding a novel acyltransferase protein from carnations and have confirmed that this gene functions in carnations. Specifically, it has been revealed that a gene encoding a protein having the activity of transferring acyl groups in carnations does not belong to a group of plant BAHD acyltransferase genes known in the art. From bicolor breeding lines having both of non-acylated anthocyanins free of acyl groups and acylated anthocyanins having acyl groups, it has been confirmed that the gene is interrupted by a transposon, and that the transposon has been excised in petals comprising acylated anthocyanins. The present inventors have further shown that the mutant gene interrupted by this transposon displays simple inheritance. The present inventors have completed the present invention by finding that a plant which has petals having a novel flower color can be produced by expressing or repressing a novel acyltransferase protein in the plant petals by use of a mutant gene of a gene encoding the novel acyltransferase protein, which comprises the transposon.

Specifically, the present invention encompasses the following inventions:
[1] A mutant gene of a gene encoding a novel acyltransferase protein, which comprises any of the following DNAs a) to d) inserted as a transposon in the nucleotide sequence of the novel acyltransferase protein-encoding gene represented by SEQ ID NO: 2:
   a) DNA comprising the nucleotide sequence represented by SEQ ID NO: 3;
   b) DNA which hybridizes under stringent conditions to DNA complementary to DNA comprising the nucleotide sequence represented by SEQ ID NO: 3 and has a transfer activity as a transposon;
   c) DNA which comprises a nucleotide sequence with 80% or higher homology to the nucleotide sequence represented by SEQ ID NO: 3 and has a transfer activity as a transposon; and
   d) DNA which comprises a degenerate isomer of the nucleotide sequence represented by SEQ ID NO: 3.
[2] The mutant gene according to [1], wherein the transposon is located within an intron between bases at the positions 732 and 733 of SEQ ID NO: 2.
[3] A plant comprising a mutant gene according to [1] or [2].
[4] A plant having a mutant gene according to [1] or [2], which has petals comprising non-acylated anthocyanins and acylated anthocyanins.
[5] The plant according to [4], wherein the plant is a progeny plant of MFA1 or MFA2 line.
[6] A process to produce a plant according to [4], comprising the following steps:
   (1) crossing a plant having a mutant gene according to [1] or [2] with a different plant comprising non-acylated anthocyanins; and
   (2) selecting an individual which has petals comprising non-acylated anthocyanins and acylated anthocyanins, from among F1 hybrids.
[7] A process to produce a progeny plant of an MFA1 or MFA2 line according to [5], comprising the following steps:
   (1) crossing an MFA1 or MFA2 line with a different plant comprising non-acylated anthocyanins; and
   (2) selecting an individual which has petals comprising non-acylated anthocyanins and acylated anthocyanins, from among F1 hybrids.
[8] The process to produce a progeny plant of an MFA1 or MFA2 line according to [7], wherein in the step (1), progeny obtained by the crossing of an MFA1 or MFA2 line is crossed with a different plant comprising non-acylated anthocyanins.
[9] A process to produce a plant according to [4], comprising the following steps:
   (1) crossing a plant having a mutant gene according to [1] or [2] with a different plant comprising acylated anthocyanins and having a gene encoding a novel acyltransferase protein or gene, one allele of which has one copy of a normal gene encoding a novel acyltransferase protein but the other allele of which has a gene encoding a novel acyltransferase protein or a mutant gene thereof which does not function; and
   (2) selecting an individual which has petals comprising non-acylated anthocyanins and acylated anthocyanins, from among F1 hybrids.
[10] A process to produce a progeny plant of an MFA1 or MFA2 line according to [5], comprising the following steps:
   (1) crossing an MFA1 or MFA2 line or progeny thereof with a different plant comprising acylated anthocyanins and having a gene encoding a novel acyltransferase protein or a mutant gene thereof, one allele of which has one copy of a normal gene encoding a novel acyltransferase protein but the other allele of which does not function; and
   (2) selecting an individual which has petals comprising non-acylated anthocyanins and acylated anthocyanins, from among F1 hybrids.
[11] The process to produce a progeny plant of an MFA1 or MFA2 line according to [10], wherein in the step (1), progeny obtained by the crossing of an MFA1 or MFA2 line is crossed with a different plant comprising non-acylated anthocyanins.

The present invention provides a plant which has petals comprising non-acylated anthocyanins and acylated anthocyanins by use of a mutant gene of a gene encoding a novel acyltransferase protein, which comprises a transposon inserted in the novel acyltransferase protein-encoding gene. A plant having the mutant gene can be bred by crossing to thereby cultivate varieties comprising non-acylated anthocyanins and acylated anthocyanins in petals and having a revolutionary variegated flower color such as a bicolor. The present invention can also detect the presence or absence of a mutation attributed to the transposon.

### BRIEF DESCRIPTION OF THE DRAWINGS

FIG. 1 is a scheme showing a reaction catalyzed by a novel acyltransferase protein. FIG 1A shows a reaction through which malic acids are added to anthocyanins having glucose at the position 3, and FIG. 1B shows a reaction through which anthocyanins having glucose at the positions 3 and 5 are converted to cyclic malyl anthocyanins.
FIG. 2 is a photograph of a flower of an MFA1 line.

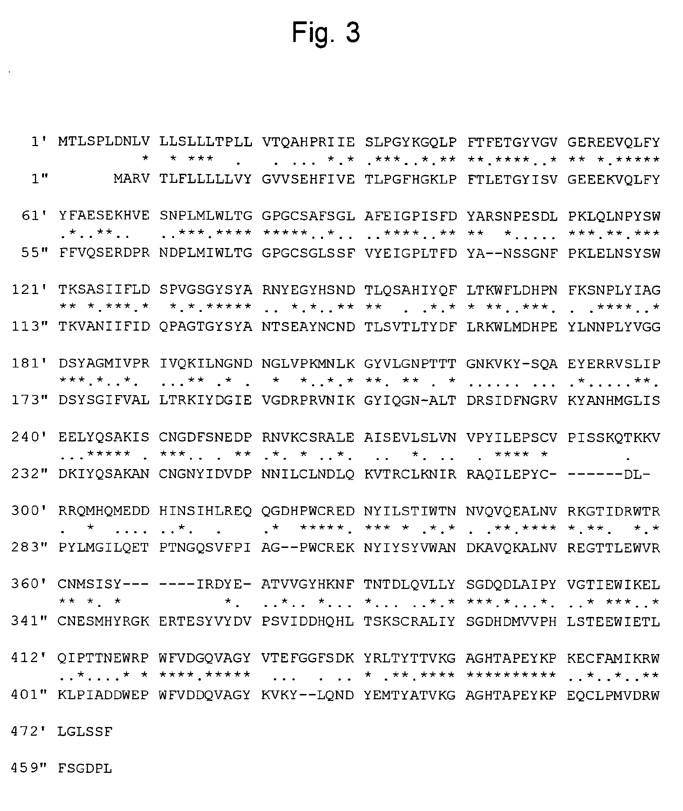

FIG. 3 is a figure showing the results of analyzing, with DNA analysis software GENETYX (ver. 4.0, Genetyx Corp.), the amino acid sequences of a protein (upper row) predicted from a cDNA sequence encoding a protein having a novel acyltransferase protein activity and a glucose/isobutyric acid transfer protein (lower row) known in the art, which synthesizes tomato acyl acetals (substances resistant to insects) and was used to isolate the novel acyltransferase protein-encoding gene. These amino acid sequences exhibit 44.6% homology as a whole, though partial more homology is observed. In the figure, the marks * and denote identical amino acids and homologous amino acids, respectively.
FIG. 4 is a figure showing an intron sequence in which a transposon has been inserted. In the figure, TSD is underlined.

FIG. 5 is a figure showing a footprint confirmed in the variegated spot of a bicolor line after the excision of the transposon. Two footprints could be confirmed for an MFA1-3 line.

FIG. 6 is a photograph of a flower of a line having petals where a base color comprises cyanidin-3,5-diglucosides, non-acylated anthocyanins, as a main pigment and is rough purple, and a variegated spot color comprises cyclic 5-3-malyl cyanidins, acylated anthocyanins, as a main pigment and is red purple.

FIG. 7 is a photograph of the petals of a flower of a line having petals where a base color comprises cyanidin-3,5-diglucosides, non-acylated anthocyanins, as a main pigment and is rough purple, and a variegated spot color comprises cyclic 5-3-malyl cyanidins, acylated anthocyanins, as a main pigment and is red purple. The base and fan-shape variegated spot of the petal can be observed.

FIG. 8 is a photograph of a flower of an HA21 line.

### DETAILED DESCRIPTION OF THE PREFERRED EMBODIMENTS

Hereinafter, the present invention will be described in detail.

### 1. A mutant gene of a gene encoding a novel acyltransferase protein

A gene of the present invention is a mutant gene of a gene encoding a novel acyltransferase protein, which comprises any of the following DNAs a) to d) inserted as a transposon at an arbitrary position of SEQ ID NO: 2:
a) DNA comprising the nucleotide sequence represented by SEQ ID NO: 3;
b) DNA which hybridizes under stringent conditions to DNA complementary to DNA comprising the nucleotide sequence represented by SEQ ID NO: 3 and has a transfer activity;
c) DNA which comprises a nucleotide sequence with 80% or higher homology to the nucleotide sequence represented by SEQ ID NO: 3 and has a transfer activity; and
d) DNA which comprises a degenerate isomer of the nucleotide sequence represented by SEQ ID NO: 3.

A gene comprising the nucleotide sequence represented by SEQ ID NO: 2 is a gene encoding a novel acyltransferase protein. The amino acid sequence of the novel acyltransferase protein encoded by the gene is shown in SEQ ID NO: 1. The novel acyltransferase protein encompasses a protein which comprises an amino acid sequence derived from the amino acid sequence represented by SEQ ID NO: 1 by the deletion, substitution, insertion, or addition of one to several amino acids and has the activity of transferring malic acids as acyl groups to anthocyanins.

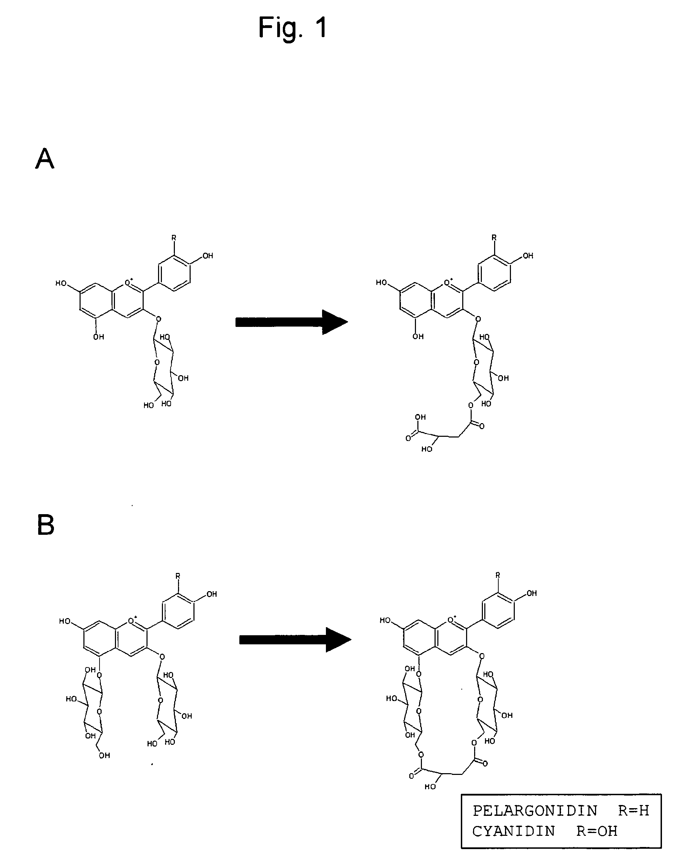

The novel acyltransferase protein having the amino acid sequence represented by SEQ ID NO: 1 has the activity of adding malic acids as acyl groups to anthocyanins having glucose at the position 3 and the activity of converting anthocyanins having glucose at the positions 3 and 5 to cyclic malyl anthocyanins. FIG 1A shows a reaction through which malic acids are added to anthocyanins having glucose at the position 3, and FIG. 1B shows a reaction through which anthocyanins having glucose at the positions 3 and 5 are converted to cyclic malyl anthocyanins.

The term "transfer activity" refers to the activity capable of being transferred on the plant genome in such a way that a fragment inserted in a gene is excised therefrom and further inserted into another gene.

Furthermore, the mutant gene of a gene comprising the nucleotide sequence represented by SEQ ID NO: 2 of the present invention encompasses, for example, DNA which comprises the nucleotide sequence represented by SEQ ID NO: 3 within an intron between bases at the positions 732 and 733 of the nucleotide sequence represented by SEQ ID NO: 2.

The range of the phrase "one to several" in the "amino acid sequence derived from the amino acid sequence represented by SEQ ID NO: 1 by the deletion, substitution, insertion, or addition of one to several amino acids" is not particularly limited and means, for example, approximately 1 to 20, preferably, approximately 1 to 10, more preferably, approximately 1 to 7, even more preferably, approximately 1 to 5, particularly preferably, approximately 1 to 3.

The deletion, substitution, insertion, or addition of amino acid(s) can be performed by modifying a gene encoding the protein by an approach known in the art. The modification can be introduced into the gene by an approach known in the art such as a Kunkel or Gapped duplex method or by a method equivalent thereto. For example, the modification is introduced thereinto by use of a modification introduction kit using a site-specific mutagenesis method (e.g., Mutant-K (manufactured by TAKARA BIO Inc.) or Mutant-G (manufactured by TAKARA BIO Inc.)) or by use of an LA PCR *in vitro* Mutagenesis series kit manufactured by TAKARA BIO Inc.

The term "novel acyltransferase protein activity" refers to the activity of adding malic acids as acyl groups to anthocyanins having glucose at the position 3 and the activity capable of converting anthocyanins having glucose at the positions 3 and 5 to cyclic malyl anthocyanins. When a certain protein has an activity substantially equivalent to the activity of the protein comprising the amino acid sequence represented by SEQ ID NO: 1, it can be said that the protein has the novel acyltransferase protein activity. In this context, the phrase "activity substantially equivalent" means that the specific activity of the enzyme is determined to be an activity with 80% or higher, preferably 90% or higher identity to the activity of the protein comprising the amino acid sequence represented by SEQ ID NO: 1.

Whether or not such a modified protein actually has the novel acyltransferase protein activity can be confirmed by: preparing a vector comprising a promoter (e.g., a cauliflower mosaic virus 35S RNA promoter) ligated downstream of the gene encoding the protein; introducing the vector into a plant accumulating therein non-acylated anthocyanins (e.g., Nazareno (Kirin Agribio Co., Ltd.) by various transformation methods (described later) that are conventionally well known and commonly used; and then measuring pigments in petals.

The mutant gene of a gene comprising the nucleotide sequence represented by SEQ ID NO: 2 encompasses, for example, DNA which hybridizes under stringent conditions to DNA complementary to DNA comprising a nucleotide sequence comprising the nucleotide sequence represented by SEQ ID NO: 3 within an intron between bases at the positions of 732 and 733 of the nucleotide sequence represented by SEQ ID NO: 2 and has the activity capable of transferring the transposon.

In this context, the stringent conditions refer to conditions under which so-called specific hybrids are formed while non-specific hybrids are not formed, and refer to, for example, conditions involving a sodium concentration of 10 mM to 300 mM, preferably 20 to 100 mM, and a temperature of 25°C to 70°C, preferably 42°C to 55°C.

The mutant gene of the present invention also encompasses DNA which comprises a nucleotide sequence with 80% or higher, more preferably 90% or higher, most preferably 95% or higher homology to a nucleotide sequence comprising the nucleotide sequence of SEQ ID NO: 3 within an intron sequence (FIG. 3) between bases at the positions of 732 and 733 of the nucleotide sequence represented by SEQ ID NO: 2 and has the activity of transferring the transposon. In this context, the homology percentage is calculated from default (initial setting) parameters by use of a nucleotide sequence comparison program (e.g., DNASIS-Mac v. 3.7 (Hitachi Software Engineering Co., Ltd.) or GENETYX ver. 4.0 (Genetyx Corp.).

The mutant gene of the present invention further encompasses a degenerate isomer thereof. In this context, the degenerate isomer means DNA that is different therefrom in degenerate codon and can encode the same protein. For example, DNA having the nucleotide sequence of SEQ ID NO: 2 but having the substitution of a codon corresponding to a certain amino acid by a codon having a degenerate relationship therewith is referred to as a degenerate isomer in the present invention. For example, DNA having the nucleotide sequence of SEQ ID NO: 2 but having the substitution of a codon (AAC) corresponding to Asn by a codon having a degenerate relationship therewith, for example, AAT, is referred to as a degenerate isomer in the present invention.

The modified gene (modified DNA) can be prepared by an approach known in the art such as a Kunkel or Gapped duplex method or by a method equivalent thereto, for example, by use of a modification introduction kit using a site-specific mutagenesis method (e.g., Mutant-K (manufactured by TAKARA BIO Inc.) or Mutant-G (manufactured by TAKARA BIO Inc.)) or by use of an LA PCR *in vitro* Mutagenesis series kit manufactured by TAKARA BIO Inc. The mutagenesis method can be selected and practiced by those skilled in the art without particular difficulties with reference to the nucleotide sequence of the gene according to the descriptions of a document such as Molecular Cloning [Sambrook et al. ed., 15, Site-directed Mutagenesis of Cloned DNA, 15.3 to 15.113, Cold Spring Harbor Lab. Press, New York (1989)]. Alternatively, techniques for artificially performing the substitution, deletion, insertion, or addition of one or more (1 or several or more) bases from the nucleotide sequence (site-specific mutagenesis) can be practiced by those skilled in the art on the basis of the nucleotide sequence of the gene according to techniques described in, for example, Proc. Natl. Acad. Sci. USA, 81, 5662-5666 (1984); WO85/00817; Nature, 316, 601-605 (1985); Gene, 34, 315-323 (1985); Nucleic Acids Res., 13, 4431-4442 (1985); Proc. Natl. Acad. Sci. USA, 79, 6409-6413 (1982); and Science, 224, 1431-1433 (1984).

The mutant gene of a gene encoding a novel acyltransferase protein of the present invention has a gene function inhibited so as not to be expressed in stems or leaves. On the other hand, its partial function can be recovered in petals by virtue of the transfer of the transposon in the mutant gene.

The mutant gene of the present invention structurally comprises a transposon by which the gene encoding a novel acyltransferase protein is interrupted. Preferably, the transposon is, for example, dTdic1 or Tdic101 capable of being transferred at a high frequency in petals. Examples of the most preferable transposon include a transposon comprising the nucleotide sequence represented by SEQ ID NO: 3. Further examples of the transposon include: DNA which hybridizes under stringent conditions to DNA complementary to DNA comprising the nucleotide sequence represented by SEQ ID NO: 3 and has a transfer activity; DNA which comprises a nucleotide sequence with 80% or higher homology to the nucleotide sequence represented by SEQ ID NO: 3 and has a transfer activity; and DNA which comprises a degenerate isomer of the nucleotide sequence represented by SEQ ID NO: 3.

A position at which the transposon interrupts the gene encoding a novel acyltransferase protein may be any of promoter, exon, intron, and noncoding regions as long as this interruption of the gene inhibits the gene function. Among them, the interruption of the intron region is preferable. The position of the intron region in the gene encoding a novel acyltransferase protein can be identified by preparing PCR primers on the basis of the nucleotide sequence of DNA encoding the novel acyltransferase protein represented by SEQ ID NO: 1, and comparing cDNA synthesized from mRNA (serving as a template) extracted from carnation petals, with the genomic DNA of carnations.

### 2. A nonfunctioning gene encoding a novel acyltransferase protein

A nonfunctioning gene encoding a novel acyltransferase protein may be a gene that does not function due to, for example, deletion or insertion in the gene of SEQ ID NO: 2 or a promoter thereof. Such a nonfunctioning gene can be tested easily by RT-PCR or PCR using primers prepared on the basis of SEQ ID NO: 2. Preferably, a nonfunctioning gene possessed as homozygous alleles by the variety 'Nazareno' (Kirin Agribio Co., Ltd.) can be utilized.

### 3. Breeding of a line which has petals comprising non-acylated anthocyanins and acylated anthocyanins

As a plant having the mutant gene of a gene encoding a novel acyltransferase protein of the present invention, which comprises an inserted transposon, is crossed with a plant free of the gene encoding a novel acyltransferase protein or having the nonfunctioning gene encoding a novel acyltransferase protein to thereby obtain, in progeny a new plant can be obtained to have the mutant gene of a gene encoding a novel acyltransferase protein of the present invention, which comprises an inserted transposon, and to have non-acylated anthocyanins and acylated anthocyanins. The plant having non-acylated anthocyanins and acylated anthocyanins has an unprecedented variegated flower color such as a bicolor. Furthermore, the crossing of progeny such as F1 hybrids can further be repeated to thereby obtain plants having a variegated flower color such as not only a bicolor but also a tricolor or tetracolor.

For example, when carnations are used as plants, gene symbols of carnations are annotated as R for flavonoid-3'-hydroxylase, r for a mutation thereof, M for flavonoid-5-glucosyltransferase, and m for a mutation thereof. Furthermore, when the gene encoding a novel acyltransferase protein is represented by Ac, a mutant gene thereof is represented by ac, and a mutant gene that displays the bicolor of a flower comprising a transposon is represented by ac-m.

For example, the genotype of a plant having the mutant gene of a gene encoding a novel acyltransferase protein of the present invention, which comprises an inserted transposon, is represented by r/r M/M ac/ac-m. The genotype of a plant free of the mutant gene of a gene encoding a novel acyltransferase protein of the present invention, which comprises an inserted transposon, is represented by r/r M/M ac/ac. The genotypes of progeny lines obtained by the crossing between both of the plants are represented by r/r M/M ac/ac-m and r/r M/M ac/ac. Of them, the progeny line represented by r/r M/M ac/ac-m is a plant which has petals having a bicolor flower color.

Examples of carnation lines having the genotype represented by r/r M/M ac/ac-m and having the mutant gene of a gene encoding a novel acyltransferase protein of the present invention, which comprises an inserted transposon, include MFA1 and MFA2 lines. Alternatively, examples of carnation lines free of the mutant gene of a gene encoding a novel acyltransferase protein of the present invention, which comprises an inserted transposon, include Nazareno.

The flower color can be determined by visual observation or can be determined by a colorimetric measurement test. In the colorimetric measurement test, the chromaticity of the petals of three individuals from each line is measured three times with a simple spectroscopic colorimeter (NF333 type) manufactured by Nippon Denshoku Industries Co., Ltd. in the fully opened state of the petals of plants that have bloomed. The average value thereof can be calculated to thereby evaluate flower colors. In this context, the colorimetric measurement is alternative means for describing the phenotypes of observed colors. It should be regarded as an index for recognized colors and does not limit obtainable potential colors.

### 4. Detection of the transposon

The nucleotide sequence of the transposon of the present invention can be utilized to thereby detect a mutant by examining a difference from the original individual by use of an approach such as southern hybridization or PCR. Specifically, the transposon in the gene encoding a novel acyltransferase protein can be detected by use of primers or probes specific to the transposon. The primers used for detecting the presence or absence of the transposon can be designed by arbitrarily selecting two portions having consecutive 8 or more bases, preferably 16 or more bases, more preferably 20 or more bases, even more preferably 20 to 30 bases, from the nucleotide sequence of the transposon. The probe that can be used for detecting the presence or absence of the transposon contains at least consecutive 20 to 100 bases selected from the nucleotide sequence of the transposon or 20 to 100 bases having a complementary sequence thereof. The probe can be labeled by means usually used, for example, radiolabeling (Amersham Megaprime) or DIG labeling (which adheres to the Roche method).

The excision of the transposon can be confirmed as follows: for example, a portion (fragment) of the mutant gene (gene comprising the inserted transposon) can be isolated by detecting the difference between the mutant and the individual serving as the origin of the mutant by Southern hybridization using the transposon obtained according to the present invention as a probe or by PCR using primers prepared on the basis of the nucleotide sequence of the transposon. The DNA fragment thus obtained can be used to easily isolate the whole mutant gene. The primers used for detecting the presence or absence of the transposon excision can be designed by arbitrarily selecting two portions having consecutive 8 or more bases, preferably 16 or more bases, more preferably 20 or more bases, even more preferably 20 to 30 bases, from each of the nucleotide sequences of the transposon and the gene encoding a novel acyltransferase protein.

### Examples

Hereinafter, the present invention will be described specifically with reference to Examples. However, the present invention is not intended to be limited to these Examples.

Carnation lines or varieties used in Examples below other than lines or varieties provided by distributors other than Kirin Agribio Co., Ltd. have been stored in Kirin Agribio Co., Ltd. and can be purchased or requested for transfer as experimental materials. Contact information: Nichibei Bldg. 8F, 2-24-2 Hacchobori, Chuo-ku, Tokyo, 104-0032, Japan, Phone: 03-5541-5875, Fax: 03-5541-5879.

### (Example 1)

Acquirement of candidate cDNA fragments encoding a novel acyltransferase protein expressed in carnation petals

The carnation variety Lucia (pink carnation as a breeding line of Kirin Agribio Co., Ltd., which comprises pelargonidin-3-malyl glucosides, acylated anthocyanins, as a main pigment) was cultivated and caused to bloom in a greenhouse according to a standard method. mRNA was prepared from petals by use of RNeasy (Qiagen). Total cDNA was synthesized by use of SuperScript First-Strand System (Invitrogen Corporation).

This cDNA was subjected to PCR (conditions: 95°C for 5 min., 30 cycles of (95°C for 30 sec., 55°C for 30 sec., and 72°C for 1 min.), and 72° for 10 min.) using primers [U592: ATGATTTGGCTTACAGGNGGNCCNGGNTG (SEQ ID NO: 4) and U593: GCTGTRTGNCCNGCNCCYTT (SEQ ID NO: 5)] prepared on the basis of the gene information of a glucose/isobutyric acid transfer protein [Li et al., PNAS, 97, 6902-6907 (2000)] synthesizing tomato acyl acetals (substances resistant to insects), a serine carboxypeptidase [Genbank ACCESSION XP_474646] having homology to the sequence thereof, which is predicted from rice genomic sequence, a barley serine carboxypeptidase [Genbank ACCESSION CAA70816] also having homology to the sequence thereof, and an *Arabidopsis* serine carboxypeptidase SNG2 [Genbank ACCESSION NP_568215] also having homology to the sequence thereof. A PCR enzyme used in this PCR and in subsequent experiments was ExTaq manufactured by Takara Shuzo Co., Ltd. The amplification products were separated by electrophoresis at 100 V for 20 minutes using a 1% agarose gel and visualized by ethidium bromide staining. The DNA fragments were confirmed to be amplified with predicted molecular weights of approximately 1 kb. The obtained amplification products were cloned by use of TOPO TA Cloning Kit for Sequencing (manufactured by Invitrogen Corporation) and sequenced for determining the nucleotide sequences by use of ABI310 (Applied Biosystems). Twenty-four nucleotide sequences were thus determined to obtain 4 types of clones, AT3-1 (13 clones), AT3-2 (9 clones), AT3-3 (1 clone), and AT3-4 (1 clone). Primers specific to each of these four types of clones were synthesized. [AT3-1: U609: GGTTGCTCTGCTTTCTCTGGCCTC (SEQ ID NO: 6) and U610: TTAACTGTAGCATAAACTAAGCGG (SEQ ID NO: 7), AT3-2: U611: GGGTGCTCTTCTTGGAACGGTCTCG (SEQ ID NO: 8) and U612: CCCTTGACTGTCGCGTACGTCAAAG (SEQ ID NO: 9), AT3-3: U622: CTGCTTTTTCTGGTTTAGCC (SEQ ID NO: 10) and U623: CAGTAGTATAGGTTAACCGG (SEQ ID NO: 11), and AT3-4: U624: GCTCTGCTTTGTCGGGCCTC (SEQ ID NO: 12) and U625: ACCGTAGCATAGACTAATCG (SEQ ID NO: 13)]

### (Example 2)

### Isolation of a gene encoding a novel acyltransferase protein

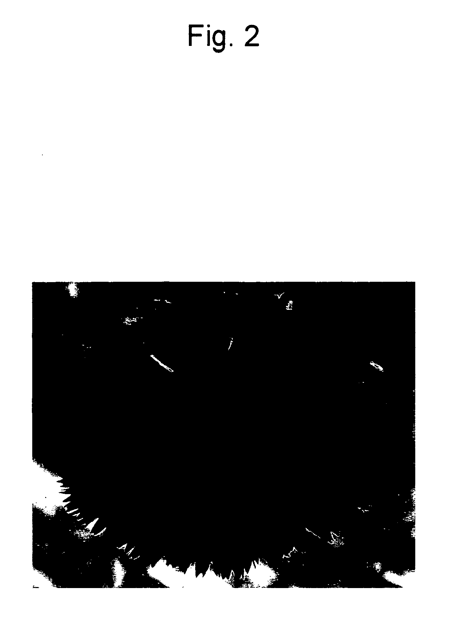

MFA1 and MFA2 lines are breeding lines of Kirin Agribio Co., Ltd. The MFA1 line (FIG. 2), its 4 progeny lines (MFA1-1 to MFA1-4, all from Kirin Agribio Co., Ltd.), the MFA2 line, and its 2 progeny lines (MFA2-1 and MFA2-2, all from Kirin Agribio Co., Ltd.) are revolutionary bicolor lines, all of which have petals where a base color is pale purple and comprises pelargonidin-3,5-diglucosides, non-acylated anthocyanins, as a main pigment, and a variegated spot color is pink and comprises cyclic 5-3-malyl pelargonidins, acylated anthocyanins, as a main pigment. Genomic DNA was extracted from leaves by use of DNeasy (Qiagen). Genomic DNA was also extracted as a control from the leaves of the carnation variety Lucia. The genomic DNAs from the leaves of the MFA1 and MFA2 lines and Lucia were subjected to genome comparison analysis using the primers synthesized in Example 1. The use of the AT3-1-specific primers (SEQ ID NOs: 6 and 7) showed that a specific insertion sequence was observed in the MFA1 line but was not inherited to the 4 progeny lines (MFA1-1 to MFA1-4). The use of the AT3-2- and AT3-4-specific primers (SEQ ID NOs: 8 and 9 and SEQ ID NOs: 12 and 13, respectively) showed that a specific insertion was not observed in the MFA1 and MFA2 lines. The use of the AT3-3-specific primers (SEQ ID NOs: 10 and 11) showed that an insertion fragment of approximately 4 kb was observed to be present in both of the MFA1 and MFA2 lines. Analysis using the same primer (AT3-3-specific primer (SEQ ID NO: 10)) showed that the insertion fragment was inherited to their respective progeny lines (MFA1-1 to MFA1-4 and MFA2-1 and MFA2-2, respectively).

To determine the full-length sequence of the AT3-3 gene, the mRNA obtained from Lucia in Example 1 was sequenced for determining the full-length cDNA sequence (SEQ ID NO: 2) by use of GeneRacer Kit (Invitrogen Corporation). FIG. 3 shows the amino acid homology between a novel acyltransferase protein (SEQ ID NO: 1) encoded by the gene and a glucose/isobutyric acid transfer protein, which synthesizes tomato acyl acetals (substances resistant to insects) and was used to isolate the novel acyltransferase protein-encoding gene. These amino acid sequences exhibit 44.6% homology as a whole, though partial more homology is observed. Most of plant acyltransferases are encoded by a group of plant BAHD acyltransferase genes described in D'Auria, Curr. Opin. Plant Biol., 9, 331-340 (2006). Surprisingly, any homology of the present gene to the group of plant BAHD acyltransferase genes was not observed.

### (Example 3)

MFA1 line- and MFA2 line-specific insertion fragments located in the gene encoding a novel acyltransferase protein

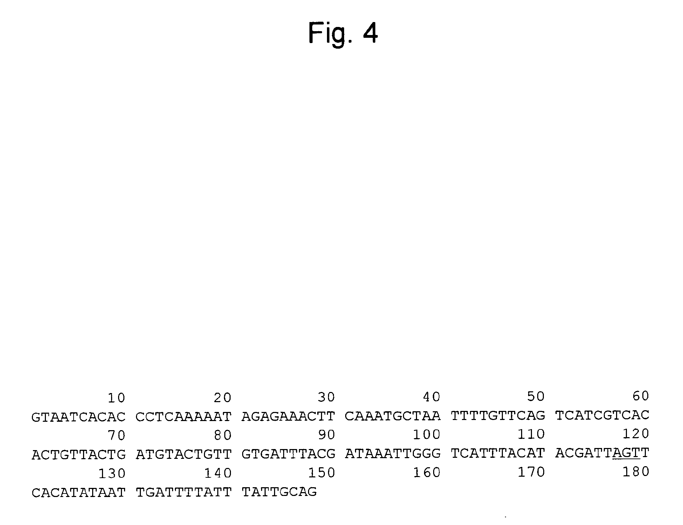

Genomic fragments from the leaves of the MFA1 and MFA2 lines were amplified by use of primers [U627: GAATATGAACGTCGCGTATCAC (SEQ ID NO: 14) and U628: CATTGCAACTGATCTTGGCCG (SEQ ID NO: 15)] prepared on the basis of the full-length cDNA sequence (SEQ ID NO: 2) of Lucia. The amplification products were cloned by use of TOPO TA Cloning Kit for Sequencing and sequenced for determining the nucleotide sequences. The insertion fragment was located within a 148-bp intron (FIG. 4) between bases at the positions 732 and 733 of the nucleotide sequence represented by SEQ ID NO: 2 and caused the duplication of AGT at the positions 117 to 119 of the sequence shown in FIG. 4. Specifically, the insertion fragment of approximately 3.6 kb was inserted therein in the form exhibiting Target site duplication (TSD) of the AGT sequence. The nucleotide sequence of the insertion fragment is shown in SEQ ID NO: 3. The same insertions were observed at the same positions of the MFA1 and MFA2 lines. The affinity between the MFA1 and MFA2 lines in terms of breeding is unknown. However, it was expected that these lines were bred from those having the same insertion fragments.

### (Example 4)

### Excision of the specific insertion fragment in a variegated spot portion

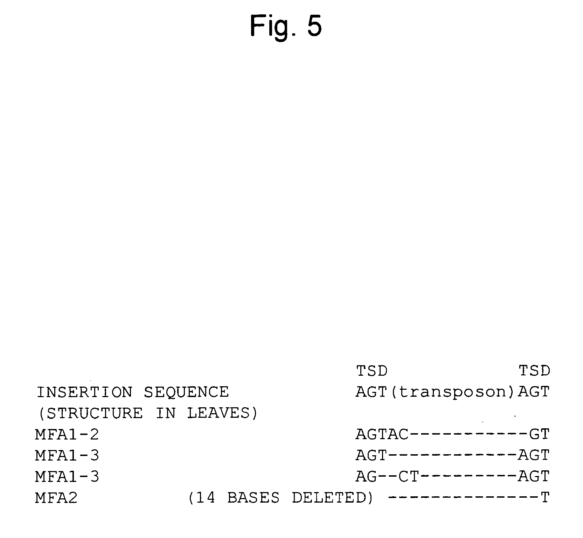

Genomic DNA was extracted from pink variegated spot portions in the petals of two progeny lines (MFA1-2 and MFA1-3) of the MFA1 line and the MFA2 line by use of DNeasy. The Genomic fragments were amplified by use of the primers (U627 and U628) used in Example 3. The amplification products were cloned by use of TOPO TA Cloning Kit for Sequencing and were determined the nucleotide sequences. As a result, the insertion fragment of Example 3 was excised from all of the petals, and the foot prints were observed (FIG. 5).

These results demonstrated that the specific insertion fragment found in Example 3 was transposon. The inserted transposon remains in leaves or in petals accumulating therein non-acylated anthocyanins. Therefore, the gene encoding a novel acyltransferase protein does not function therein, resulting in the accumulation of non-acylated anthocyanins. On the other hand, the transposon has been excised in variegated spots accumulating therein acylated anthocyanins. As a result, it was demonstrated that the gene encoding an acyltransferase protein functions therein, resulting in conversion to acylated anthocyanins. As a result, plants having a bicolor flower color can be obtained.

### (Example 5)

### Breeding of a line which has petals comprising non-acylated anthocyanins and acylated anthocyanins

A 03-28-2 line (Kirin Agribio Co., Ltd.) is pot-type carnations having pale purple petals comprising pelargonidin-3,5-diglucosides, non-acylated anthocyanins, as a main pigment. Plants having pale purple petals comprising pelargonidin-3,5-diglucosides, non-acylated anthocyanins, as a main pigment, as with the 03-28-2 line as well as plants having bicolor petals, as with the MFA1 line were obtained from seeds of the MFA1 line crossed with the 03-28-2 line. Likewise, plants having pale purple petals comprising pelargonidin-3,5-diglucosides, non-acylated anthocyanins, as a main pigment, as with the 03-28-2 line as well as plants having bicolor petals, as with the MFA1 line were obtained from seeds of the MFA2 line crossed with the 03-28-2 line. DNA was extracted from 20 plants in total (5 plants each) of these 4 types of plants or lines and examined for the inheritance of the insertion sequence by use of the primers U627 and U628 shown in Example 3. As a result, it was shown that the gene having the insertion sequence, that is, the recessive mutant gene, was inherited to the individuals or line having a bicolor flower color. These results demonstrated that the mutant gene that displays the bicolor property displays a simple recessive inheritance property.

Hereinafter, principles thereof and a process to produce a bicolor variety will be shown in detail by use of gene symbols. Gene symbols of carnations are annotated as R for flavonoid-3'-hydroxylase, r for a mutation thereof, M for flavonoid-5-glucosyltransferase, and m for a mutation thereof [Mehlquist (1939) Proc. Am. Soc. Hort. Sci 37, 1019-1021 (1939)]. Moreover, the symbol Ac is used for representing the gene encoding a novel acyltransferase protein according to the prediction of Yamaguchi et al. [Journal of Horticulture (Engeigaku Zasshi in Japanese), 69 suppl. 1, 352 (2000)]. A mutant gene thereof is represented by ac, and a mutant gene that displays the bicolor of a flower comprising a transposon is represented by ac-m. The genotypes of the MFA1 and MFA2 lines are r/r M/M ac/ac-m. On the other hand, the genotype of the pot-type carnation 03-28-2 line is r/r M/M ac/ac. Accordingly, their progeny appears at an r/r M/M ac/ac-m-to-r/r M/M ac/ac ratio of approximately 1:1. All the genotypes of the bicolor progeny lines (MFA1-1 to MFA1-4, MFA2-1, MFA2-2, etc.) were r/r M/M ac/ac-m. In this way, bicolor lines can be produced which have petals where a base color is pale purple and comprises pelargonidin-3,5-diglucosides, non-acylated anthocyanins, as main pigments, and a variegated spot color is pink and comprises cyclic 5-3-malyl pelargonidins, acylated anthocyanins, as a main pigment. Lines having the mutant gene ac-m of the present invention derived from the MFA1 or MFA2 line can be crossed as one parent to thereby produce bicolor lines. In this case, the other parent does not have to be limited to the 03-28-2 line. For example, the variety 'Nazareno' can be utilized which has pale purple petals comprising pelargonidin-3,5-diglucosides, non-acylated anthocyanins, as a main pigment. When the variety 'Mystic Barbara' (Hilverda) is utilized which has pale purple petals comprising non-acylated anthocyanins as a main pigment but is a peripheral chimera having Ac/ac as the genotype of the inner layer, the expected individuals having a bicolor flower color appear, albeit at a low frequency, in progeny and can be obtained. Even carnations that do not have any ac mutation can be used as the other parent to obtain, in progeny, lines having a bicolor flower color as long as the lines are selected from the generation after the next.

### (Example 6)

Breeding of a further new line which has petals comprising non-acylated anthocyanins and acylated anthocyanins

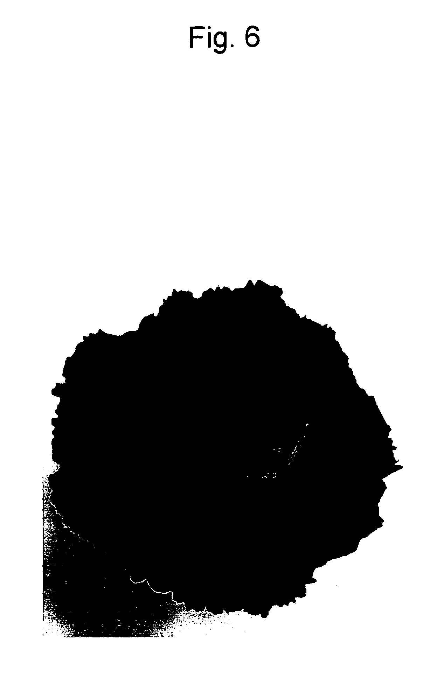

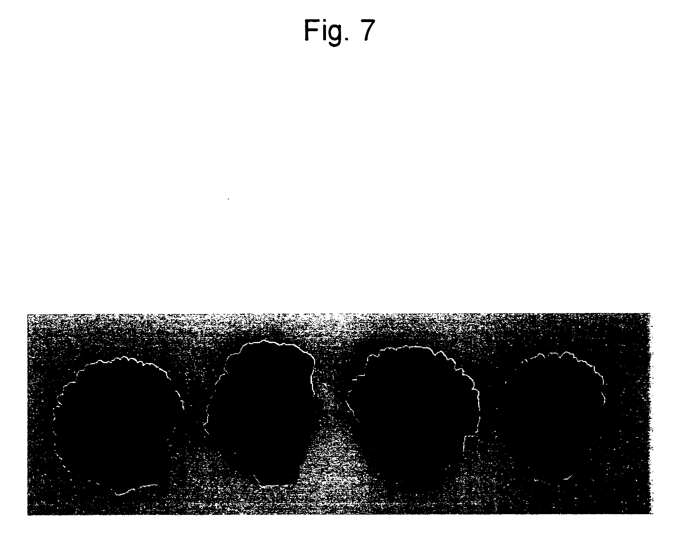

To obtain a new flower color, an HA2 line (Kirin Agribio Co., Ltd.; genotype: R/r M/M ac/ac, L*a*b* colorimetric system (Japan Industrial Standard JIS Z 8729): L* = 28.81, a* = 18.72, and b* = -8.75)) having petals where a base color is rough purple and comprises cyanidin-3,5-diglucosides, non-acylated anthocyanins, as a main pigment was crossed with the MFA2 line. As a result, lines were successfully produced which had petals where a base color is rough purple and comprises cyanidin-3,5-diglucosides, non-acylated anthocyanins, as a main pigment, and a variegated spot color is red purple and comprises cyclic 5-3-malyl cyanidins, acylated anthocyanins, as a main pigment (FIGS. 6 and 7; L*a*b* colorimetric system of the non-acylated anthocyanin portion in the base: L* = 38.22, a* = 21.33, and b* = -9.99, L*a*b* colorimetric system of the acylated anthocyanin portion in the variegated spot: L* = 32.22, a* = 53.16, and b* = -9.01). In the colorimetric measurement test, the chromaticity of the petals of three individuals from each line is measured three times with a simple spectroscopic colorimeter (NF333 type) manufactured by Nippon Denshoku Industries Co., Ltd. in the fully opened state of the petals of the carnations that have bloomed. The average value thereof can be calculated to thereby evaluate flower colors. In this context, the colorimetric measurement is alternative means for describing the phenotypes of observed colors. It should be regarded as an index for recognized colors and does not limit obtainable potential colors. The genotypes of lines obtained by the crossing between the HA2 and MFA2 lines are R/r M/M ac/ac-m. For example, the variety 'Ribe' (H. Yoshida, Journal of Horticulture (Engeigaku Zasshi in Japanese), 72 suppl. 1, 130 (2003)) can be used as one parent instead of the HA2 line.

While plants are selected in the F2 generation after the crossing between the MFA1 or MFA2 line and a different line, the crossing of the MFA1 or MFA2 line with a carnation having petals comprising pelargonidin-3-glucosides, non-acylated anthocyanins, as a main pigment, can produce lines having petals where a base portion comprises pelargonidin-3-glucosides, non-acylated anthocyanins, as main pigments, and a variegated spot portion comprises pelargonidin-3-malyl glucosides, acylated anthocyanins, as a main pigment. For example, the variety 'Cassis' (Daiichi Engei Co., Ltd.) (H. Yoshida, Journal of Horticulture (Engeigaku Zasshi in Japanese), 72 suppl. 1, 130 (2003)) can be used as one parent in this crossing. The genotypes of bicolor lines obtained by the crossing between the MFA1 or MFA2 line and 'Cassis' are r/r m/m ac/ac-m and r/r m/m ac-m/ac-m.

Alternatively, while plants are selected in the F2 generation after the crossing between the MFA1 or MFA2 line and a different line, the crossing of the MFA1 or MFA2 line with a carnation having petals comprising cyanidin-3-glucosides, non-acylated anthocyanins, as a main pigment, can produce lines having petals where a base portion comprises cyanidin-3-glucosides, non-acylated anthocyanins, as a main pigment, and a variegated spot portion comprises cyanidin-3-malyl glucosides, acylated anthocyanins, as a main pigment. For example, the variety 'Brick' (H. Yoshida, Journal of Horticulture (Engeigaku Zasshi in Japanese), 72 suppl. 1, 130 (2003)) can be used as one parent in this crossing. The genotypes of bicolor lines obtained by the crossing between the MFA1 or MFA2 line and 'Brick' are R/r m/m ac/ac-m, R/R m/m ac/ac-m, R/r m/m ac-m/ac-m, and R/R m/m ac-m/ac-m.

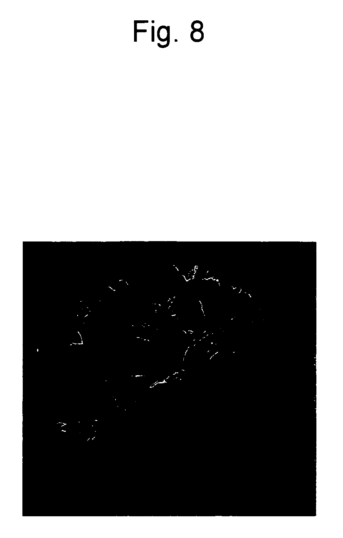

Furthermore, a combination with an inheritance mode of a known bicolor can produce tricolor or tetracolor varieties that have been unknown as carnations and are exceedingly rare in terms of the horticulture of flowering plants. The genotype of an HA21 line shown in FIG. 8 is r/r M/M ac/ac i/i-m. In this context, the symbols I and i denote a chalcone isomerase and a mutant gene, respectively. The symbol i-m denotes a gene that displays streaks seen in FIG. 8. This gene is different from genes represented by ac or ac-m and is independently inherited. A combination with the mutant gene ac-m of the MFA1 or MFA2 line (r/r M/M ac/ac-m I/I) can produce tetracolor lines (genotype: r/r M/M ac/ac-m i/i-m, base color: pale vermilion including chalcone and pelargonidin-3,5-diglucosides, color of variegated spots formed therein: yellowish pink including cyclic 5-3 malyl pelargonidins; streaks are pale purple and comprises pelargonidin-3,5-diglucosides, non-acylated anthocyanins, as main pigments, and the portion where the streak and the variegated spot overlap with each other has a pink color including cyclic 5-3 malyl pelargonidin). In addition to the HA21 line, lines having red streaks on a base yellow color such as the variety 'Kirikaoren' (Kirin Agribio Co., Ltd., genotype: r/r m/m Ac/Ac i-m/i-m) can be utilized as parents for these lines.

A gene (mutant gene) of the present invention, which comprises an inserted transposon and a process to produce a plant by use of the same can expand the flower color range of flowering plants and are useful in the development of ornamental plants. Moreover, the detection of the transposon can determine a mutation in flower colors.

## Claims

1. A mutant gene of a gene encoding a novel acyltransferase protein, which comprises any of the following DNAs a) to d) inserted as a transposon in the nucleotide sequence of the novel acyltransferase-encoding gene represented by SEQ ID NO: 2:
a) DNA comprising the nucleotide sequence represented by SEQ ID NO: 3;
b) DNA which hybridizes under stringent conditions to DNA complementary to DNA comprising the nucleotide sequence represented by SEQ ID NO: 3 and has a transfer activity as a transposon;
c) DNA which comprises a nucleotide sequence with 80% or higher homology to the nucleotide sequence represented by SEQ ID NO: 3 and has a transfer activity as a transposon; and
d) DNA which comprises a degenerate isomer of the nucleotide sequence represented by SEQ ID NO: 3.

2. The mutant gene according to claim 1, wherein the transposon is located within an intron between bases at the positions 732 and 733 of SEQ ID NO: 2.

3. A plant comprising a mutant gene according to claim 1 or 2.

4. A plant having a mutant gene according to claim 1 or 2, which has petals comprising non-acylated anthocyanins and acylated anthocyanins.

5. The plant according to claim 4, wherein the plant is a progeny plant of an MFA1 or MFA2 line.

6. A process to produce a plant according to claim 4, comprising the following steps:
(1) crossing a plant having a mutant gene according to claim 1 or 2 with a different plant comprising non-acylated anthocyanins; and
(2) selecting an individual which has petals comprising non-acylated anthocyanins and acylated anthocyanins, from among F1 hybrids.

7. A process to produce a progeny plant of an MFA1 or MFA2 line according to claim 5, comprising the following steps:
(1) crossing an MFA1 or MFA2 line with a different plant comprising non-acylated anthocyanins; and
(2) selecting an individual which has petals comprising non-acylated anthocyanins and acylated anthocyanins, from among F1 hybrids.

8. The process to produce a progeny plant of an MFA1 or MFA2 line according to claim 7, wherein in the step (1), progeny obtained by the crossing of an MFA1 or MFA2 line is crossed with a different plant comprising non-acylated anthocyanins.

9. A process to produce a plant according to claim 4, comprising the following steps:
(1) crossing a plant having a mutant gene according to claim 1 or 2 with a different plant comprising acylated anthocyanins and having a gene encoding a novel acyltransferase protein gene, one allele of which has one copy of a normal gene encoding a novel acyltransferase protein but the other allele of which has a gene encoding a novel acyltransferase protein or a mutant gene thereof which does not function; and
(2) selecting an individual which has petals comprising non-acylated anthocyanins and acylated anthocyanins, from among F1 hybrids.

10. A process to produce a progeny plant of an MFA1 or MFA2 line according to claim 5, comprising the following steps:
(1) crossing an MFA1 or MFA2 line or progeny thereof with a different plant comprising acylated anthocyanins and having a gene encoding a novel acyltransferase protein or a mutant gene thereof, one allele of which has one copy of a normal gene encoding a novel acyltransferase protein but the other allele of which does not function; and
(2) selecting an individual which has petals comprising non-acylated anthocyanins and acylated anthocyanins, from among F1 hybrids.

11. The process to produce a progeny plant of an MFA1 or MFA2 line according to claim 10, wherein in the step (1), progeny obtained by the crossing of an MFA1 or MFA2 line is crossed with a different plant comprising non-acylated anthocyanins.


SEQUENCE LISTING

<110>  KIRIN HOLDINGS KABUSHIKI KAISHA

<120>  A process to produce a plant which has petals comprising
       non-acylated anthocyanins and acylated anthocyanins

<130>  PH-3405EP

<150>  JP2006-356440
<151>  2006-12-28


<160>  15

<170>  PatentIn version 3.4

<210>  1
<211>  477
<212>  PRT
<213>  Dianthus caryophyllus

<400>  1

Met Thr Leu Ser Pro Leu Asp Asn Leu Val Leu Leu Ser Leu Leu Leu
1               5                   10                  15


Thr Pro Leu Leu Val Thr Gln Ala His Pro Arg Ile Ile Glu Ser Leu
            20                  25                  30


Pro Gly Tyr Lys Gly Gln Leu Pro Phe Thr Phe Glu Thr Gly Tyr Val
        35                  40                  45


Gly Val Gly Glu Arg Glu Glu Val Gln Leu Phe Tyr Tyr Phe Ala Glu
    50                  55                  60


Ser Glu Lys His Val Glu Ser Asn Pro Leu Met Leu Trp Leu Thr Gly
65                  70                  75                  80

Gly Pro Gly Cys Ser Ala Phe Ser Gly Leu Ala Phe Glu Ile Gly Pro
                85                  90                  95

Ile Ser Phe Asp Tyr Ala Arg Ser Asn Pro Glu Ser Asp Leu Pro Lys
            100                 105                 110

Leu Gln Leu Asn Pro Tyr Ser Trp Thr Lys Ser Ala Ser Ile Ile Phe
        115                 120                 125

Leu Asp Ser Pro Val Gly Ser Gly Tyr Ser Tyr Ala Arg Asn Tyr Glu
    130                 135                 140

Gly Tyr His Ser Asn Asp Thr Leu Gln Ser Ala His Ile Tyr Gln Phe
145                 150                 155                 160

Leu Thr Lys Trp Phe Leu Asp His Pro Asn Phe Lys Ser Asn Pro Leu
                165                 170                 175

Tyr Ile Ala Gly Asp Ser Tyr Ala Gly Met Ile Val Pro Arg Ile Val
            180                 185                 190

Gln Lys Ile Leu Asn Gly Asn Asp Asn Gly Leu Val Pro Lys Met Asn
        195                 200                 205

Leu Lys Gly Tyr Val Leu Gly Asn Pro Thr Thr Thr Gly Asn Lys Val
    210                 215                 220

Lys Tyr Ser Gln Ala Glu Tyr Glu Arg Arg Val Ser Leu Ile Pro Glu
225                 230                 235                 240

Glu Leu Tyr Gln Ser Ala Lys Ile Ser Cys Asn Gly Asp Phe Ser Asn
                245                 250                 255

Glu Asp Pro Arg Asn Val Lys Cys Ser Arg Ala Leu Glu Ala Ile Ser
            260                 265                 270

Glu Val Leu Ser Leu Val Asn Val Pro Tyr Ile Leu Glu Pro Ser Cys
        275                 280                 285

Val Pro Ile Ser Ser Lys Gln Thr Lys Lys Val Arg Arg Gln Met His
    290                 295                 300

Gln Met Glu Asp Asp His Ile Asn Ser Ile His Leu Arg Glu Gln Gln
305                 310                 315                 320

Gly Asp His Pro Trp Cys Arg Glu Asp Asn Tyr Ile Leu Ser Thr Ile
                325                 330                 335

Trp Thr Asn Asn Val Gln Val Gln Glu Ala Leu Asn Val Arg Lys Gly
            340                 345                 350

Thr Ile Asp Arg Trp Thr Arg Cys Asn Met Ser Ile Ser Tyr Ile Arg
        355                 360                 365

Asp Tyr Glu Ala Thr Val Val Gly Tyr His Lys Asn Phe Thr Asn Thr
    370                 375                 380

Asp Leu Gln Val Leu Leu Tyr Ser Gly Asp Gln Asp Leu Ala Ile Pro
385                 390                 395                 400

Tyr Val Gly Thr Ile Glu Trp Ile Lys Glu Leu Gln Ile Pro Thr Thr
                405                 410                 415

Asn Glu Trp Arg Pro Trp Phe Val Asp Gly Gln Val Ala Gly Tyr Val
            420                 425                 430

Thr Glu Phe Gly Gly Phe Ser Asp Lys Tyr Arg Leu Thr Tyr Thr Thr
        435                 440                 445

Val Lys Gly Ala Gly His Thr Ala Pro Glu Tyr Lys Pro Lys Glu Cys
    450                 455                 460

Phe Ala Met Ile Lys Arg Trp Leu Gly Leu Ser Ser Phe
465                 470                 475

<210>  2
<211>  1434
<212>  DNA
<213>  Dianthus caryophyllus

<400>  2
atgacacttt cgccgcttga taatttggtg cttttgtctc ttttattaac accgttactg      60

gttacacaag ctcacccacg tattatcgag tctctgcctg gttataaagg ccagcttccc     120

ttcacattcg aaactggata tgtaggtgtt ggagaaagag aggaagtgca gttattctat     180

tactttgcgg aatcagaaaa acatgttgaa agtaatccgc ttatgctttg gctcactggt     240

ggccctggtt gctctgcttt ttctggttta gcctttgaaa ttggtccaat atcatttgat     300

tatgcgagaa gcaatccaga atcagactta ccaaagcttc agttaaatcc atattcatgg     360

acaaagtcgg ctagcattat ttttctcgat tcaccggtgg ggtcaggcta ctcgtacgcc     420

aggaactacg agggatatca cagtaatgac actttgcagt ccgcccacat ttaccagttt     480

ttgactaagt ggtttctgga ccatccaaat ttcaaaagca atcctttgta cattgctggc     540

gattcatatg cgggaatgat tgtccccagg attgtgcaaa agatcttaaa tgggaatgac     600

aatggtcttg taccaaaaat gaatctgaag ggttatgttc tcggcaatcc aacgaccaca     660

ggaaataaag tcaaatattc acaagctgaa tatgaacgtc gcgtatcact catacctgag     720

gaactctacc agtcggccaa gatcagttgc aatggtgatt tttcaaatga ggatccaaga     780

aatgtaaagt gctcacgagc gcttgaagcc atatctgaag ttctaagttt ggttaacgta 840

ccatatatcc tggagccaag ttgtgtaccg atctcttcaa agcaaactaa gaaagttcga 900

agacagatgc atcagatgga agatgatcac atcaattcta ttcatttgcg tgagcagcaa 960

ggtgaccatc catggtgtcg agaagataat tacattcttt caaccatctg gactaacaat 1020

gtccaagttc aagaagcact caacgtccga aagggaacaa tagatcgttg gacgagatgt 1080

aatatgagca tatcgtacat cagggattat gaagccactg ttgtagggta ccataaaaat 1140

ttcacaaata cagatctcca agtactgctg tacagtggtg atcaagacct cgccatacct 1200

tatgttggaa caatagaatg gatcaaagag ctacaaatcc caaccacaaa cgaatggaga 1260

ccatggtttg ttgatggtca agttgccgga tacgtcacag agtttggagg cttctcggat 1320

aaataccggt taacctatac tactgttaag ggagctggac atactgctcc tgagtataag 1380

cccaaagagt gttttgcaat gatcaaaagg tggcttggat tatcctcttt ctag 1434

<210> 3
<211> 3646
<212> DNA
<213> Dianthus caryophyllus

<400> 3
cactacaaga aaatcttgta caggcgaccg aaaaaggcta ccaacaacag tcgctaaaac 60

aagcgggcga ctgactgtag tcgccagaca gcggtagcga ggctcagtcg ccattttttg 120

aggtcgggcg accgaaatca gtcgccaaat attggcgacc gacatatcag tcgccaaaaa 180

gggggcgggc gaccgacatc agtcgcccca tgtttcccct tttttaaaaa aaaaatgggg 240

gttggcgacc gacttcggtc gccatttatg aggcgggcga ccgcaagtcg gtcgccaggc 300

agtcgccatt tctctgtttt ttctaaattt ttgtaggatt cggcgaccga taatcagtcg 360

ccaccttaat atttcagtcg ccaaactact gcaaatttac aattgctgca cggtttcacg 420

ttttgcccgt attaatttca ttaccaaatt cagcaaacct gcaaattcaa cggtataacc 480

aacccataca atttcaacaa acagcataaa caaccattca aattcaacat tttcaaacag 540

aaattaagtt tcatacatgc aagtttagta tgcaacacat aattcaagta aacatgttca 600

acatccgcta acagtttgca taaacggaaa aagagatact ttacacttga aacccaacag 660

atgctccact actcgggcca ccaccatgag ggtctctagg gtcaggaaac tgcggaccaa 720

tccctggatt acactgctca aagtaggtct gcatccgctg caattgttcc ctcatcaacc 780

tgagctcctc gtcttgagca gaaaggcgtt cgtcttgatt ggcaacgcga tcttctaatt 840

tccttacaat acccggcgtg tacatggagt cgctcactgt cgacaatctg cggtttctct 900

tctcgtaaat atactcggct gcgttgccga ctccataaac cgcccctttt ttaaaccctt 960

cgttcacttc caaccaaatt tgatcgggtt tagttccagg ctcctcctcg actcgcttca 1020

tatacgcctc ctaccaaaga gcaaatcaat tatgctattt aattacaaca aattaaaaag 1080

attataaatc tcattacgac caacaattcc ttacatattt atccgagtct ttctttctcg 1140

cgaaatgcaa acggccttcc tcgtcttttt tggcgtgcgt gtccaaaaaa tgtcaaacgc 1200

ggtcgccgct tgtcctttgt ttgcctttag tccaaaaaac ggcaacaatt atccatttaa 1260

atatacacaa tcatactata tattattgga ttatgtaaac gatatattaa aacttacttt 1320

ttctagttct tgtgcacgat caaggcagct ctgagatcct ctaaagtaac tcggctcgac 1380

gcgcgcattt ggatctcctc ccctaatatt caacgccgct tgtttggatt tcctttgaaa 1440

ttccggtgtg tctcgtaact taagcaagtc gttgaatgca acatctacaa gaaaaaatat 1500

gacgacaacc aaataaaacc gaacaacaaa ttgccgaaat atcaatttta taacctcatt 1560

tttattaaag ttagttttat taccattcat gaatgcgggc ttcttctttc ttttgacagc 1620

cttccacatg ttgtccttga agcgtttttg acacgttttc tcgtactttg cacggactaa 1680

atgctcaaga accggattcc aagtgcacga attctacgta taaaaccaaa tattaaacaa 1740

attccggcgt aataatatat atacatttta aaaacactag tggctttaaa aattatatac 1800

gtacccggaa aatattaaac cactgctcct tcaagtcgtc tggtatcgac gaccaacaag 1860
tgtaaccgga agtgtaactg ctttttgtac tctccgtaca tgccttagta attttctttt 1920
gcggaaacct tacaattttt gcgttaaaat aataagcaat atgaacaact taccaatatg 1980
attaacttac caatataaac aacttaccaa tatgatttcg ggtctaaaat taatttgccg 2040
gtcgcatctg ttgggggggt gtcgtccgcc tcctgctctt cgttttgttg ttgcgtctcc 2100
tgactatccg acgactcagt ctcctcgtcg gacctcattg acgatctgga ggaactacct 2160
tttctgcttc gccctgacga cataattcaa actaaaaaaa aaacaacatt aacggggcct 2220
attattaaat gagaacaagt tagtaacaca ctactaactt gtcaaatctt tcctcataca 2280
tcccattcgc gctccaatct cttcataata ggtcgttttc tcttatcaaa tctttcctca 2340
tacatccatt cgcgctccaa tctcttcatt attataagct aaccaatata agagaaataa 2400
ttcaataccc aaaagttcaa acaagtaatt taaccaactt aatatttgag aactaatttc 2460
aacttaatat ttgagaactt aacaaaaaaa cctcaaactc acaatcccta aattttattg 2520
aaatcaatcc tacattaata gaaaataata acactaatct taatcaaaac atattctaac 2580
ataaaaataa taatctaaca ttatctaatc ttacataaca tttaaacatt ctctaatctt 2640
aaactatcat tcaaacattc tctaatctta aataaattct ctaatataca cattggtgta 2700
tgaggctaaa tccaaccaac aacatcaaag aatagaagct tatttaggct ccaccaacca 2760
acatccaacc aaccaacaac atcaaattct ctaatataca aatgctcaaa tcaaaaattc 2820
tctcatgtaa gctccaccaa caacatcaaa ccacactatt aaggccaatt caaagcttat 2880
ttaggctatt atatcaaact taactcaaat tagaaacttc aagaatacac aaatgtcaaa 2940
accgctcctc tacgaatccg tgctctacaa ataaaccatt ctactatcaa ataccatctt 3000
aaaactacct aattaacaca ctaattcaat aaaatcggct ataaatcgaa aacccctaat 3060
ttctagggtt agggtttgac gaaaattaaa ttaaaacgat taactacgaa tcataccttt 3120

aacaactcga ttaactaggc tagaacttca aatcccgaca aaaacaacca aaagtcgacc 3180

caaaaacggc cccaacgaaa acagcggaga agatggtggt gcgtgaatgg tggtgatgcg 3240

gtggcggtgg tgcggtgacg gcggcacggt gcggtgttgg tggtgcgtga atggtggtga 3300

tggtggtgga tgatgaatga agatggtggt tgatgaagac ggtggttgca ctactcttaa 3360

tctccttttt tttttttttt tttctaagta taatcgaaag agtaagttag ggggagaagc 3420

tgcggatgaa tctaaaatgt ggggcgactg aattggcgac cgaagtcggt cgcccgatat 3480

ttttttggcg accgaagtcg gtcgcccact tatttttttt tagtcgcccc ttagtcgcct 3540

ttggtcgact aatttcagtc gcccgatcaa tatttcggtc gcctgttagc gactacatta 3600

ttcagtcgcc attttacggt cgcccgtaat agattttctt gtagtg 3646

<210> 4
<211> 29
<212> DNA
<213> Artificial

<220>
<223> Primer


<220>
<221> misc_feature
<222> (18)..(18)
<223> n is a, c, g, or t

<220>
<221> misc_feature
<222> (21)..(21)
<223> n is a, c, g, or t

<220>
<221> misc_feature
<222> (24)..(24)
<223> n is a, c, g, or t

<220>
<221> misc_feature
<222> (27)..(27)
<223> n is a, c, g, or t

<400> 4
atgatttggc ttacaggngg nccnggntg 29

<210> 5
<211> 20
<212> DNA
<213> Artificial

<220>
<223> Primer

<220>
<221> misc_feature
<222> (9)..(9)
<223> n is a, c, g, or t

<220>
<221> misc_feature
<222> (12)..(12)
<223> n is a, c, g, or t

<220>
<221> misc_feature
<222> (15)..(15)
<223> n is a, c, g, or t

<400> 5
gctgtrtgnc cngcnccytt 20

<210> 6
<211> 24
<212> DNA
<213> Artificial

<220>
<223> Primer

<400> 6
ggttgctctg ctttctctgg cctc 24

<210> 7
<211> 24
<212> DNA
<213> Artificial

<220>
<223> Primer

<400> 7
ttaactgtag cataaactaa gcgg 24

<210> 8
<211> 25
<212> DNA
<213> Artificial

<220>
<223> Primer

<400> 8
gggtgctctt cttggaacgg tctcg 25

<210> 9
<211> 25
<212> DNA
<213> Artificial

<220>
<223> Primer

<400> 9
cccttgactg tcgcgtacgt caaag 25

<210> 10
<211> 20
<212> DNA

<213>  Artificial

<220>
<223>  Primer

<400>  10
ctgctttttc tggtttagcc                                                    20


<210>  11
<211>  20
<212>  DNA
<213>  Artificial

<220>
<223>  Primer

<400>  11
cagtagtata ggttaaccgg                                                    20


<210>  12
<211>  20
<212>  DNA
<213>  Artificial

<220>
<223>  Primer

<400>  12
gctctgcttt gtcgggcctc                                                    20


<210>  13
<211>  20
<212>  DNA
<213>  Artificial

<220>
<223>  Primer

<400>  13
accgtagcat agactaatcg                                                    20

<210> 14
<211> 22
<212> DNA
<213> Artificial

<220>
<223> Primer

<400> 14
gaatatgaac gtcgcgtatc ac 22

<210> 15
<211> 21
<212> DNA
<213> Artificial

<220>
<223> Primer

<400> 15
cattgcaact gatcttggcc g 21